(19) Europäisches Patentamt
European Patent Office
Office européen des brevets

(11) Publication number: 0 248 916 A1

# (12) EUROPEAN PATENT APPLICATION

(21) Application number: 86107692.5

(22) Date of filing: 05.06.86

(51) Int. Cl.[4]: C07D 313/00, B01D 15/08, //A23K1/16,A61K31/365

(43) Date of publication of application: 16.12.87 Bulletin 87/51

(84) Designated Contracting States: AT BE CH DE FR GB LI LU NL SE

(71) Applicant: C.R.C. Compagnia di Ricerca Chimica S.p.A.
Via Pesenalat, 6
I-33048 San Giovanni al Natisone (Udine)(IT)

(72) Inventor: Decorte, Enio, Dr.
Via Zorutti, 3
Aiello del Friuli (UD)(IT)
Inventor: Moimas, Flavio, Dr.
Via D'Annunzio 81/A
Ronchi dei Legionari (GO)(IT)
Inventor: Angeli, Cristina, Dr.
Viale Trieste 166
Udine(IT)

(74) Representative: Bartels, Hans et al
Lange Strasse 51
D-7000 Stuttgart 1(DE)

## (54) Method for separation of 3S,7R-3,4,5,6,7,8,9,10,11,12-decahydro-7,14,16-trihydroxy-3-methyl-1H-2-benzoxacyclotetradecin-1-one from its 3S,7S-diastereomer.

(57) A method for the separation of 3S,7R-3,4,5,6,7,8,9,10,11,12-decahydro-7,14,16-trihydroxy-3-methyl-1H-2-benzoxacyclotetradecin-1-one from its 3S,7S-diastereomer is disclosed. In said method a mixture of the two diastereomers is dissolved in a solvent, the resulting solution is applied to a high pressure liquid chromatograph comprising a reverse-phase column, the two diastereomers are eluted with a mixture of polar solvents and the diastereomers are separately recovered.

Fig. 2

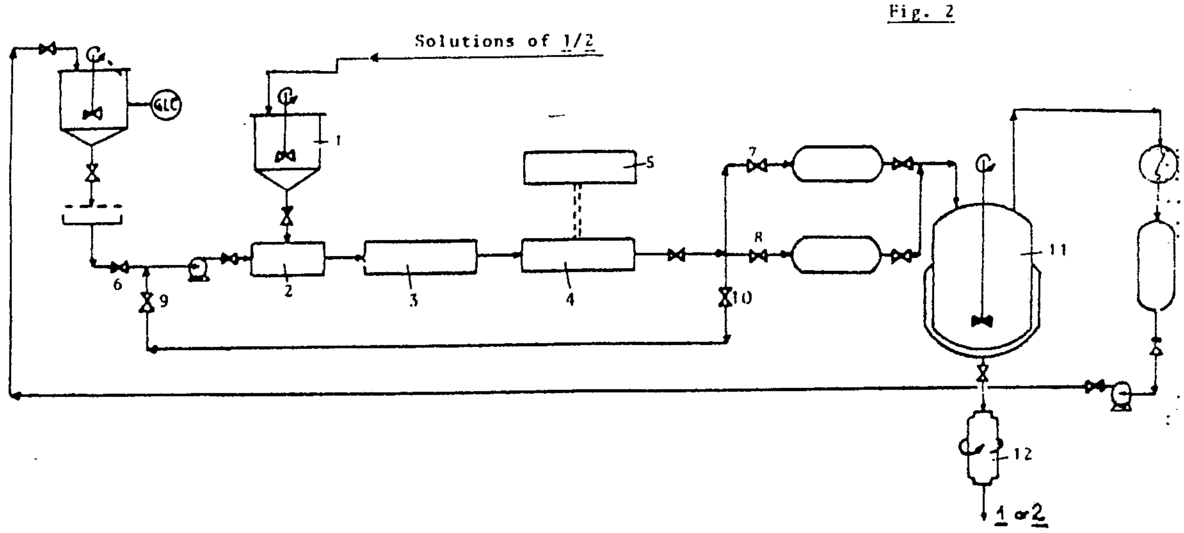


EP 0 248 916 A1

# Method for Separation of 3S, 7R-3,4,5,6,7,8,9,10,11,12-Decahydro-7,14,16-trihydroxy-3-methyl-1H-2-benzoxacyclotetradecin-1-one from its 3S,7S-Diastereomer

## Description of the Invention:

The invention relates to a method for the separation of 3S,7R-3,4,5,6,7,8,9,10,11,12-decahydro-7,14,16-trihydroxy-3-methyl-1H-2-benzoxacyclotetradecin-1-one ( 1) from its 3S,7S-diastereomer ( 2),

(1)

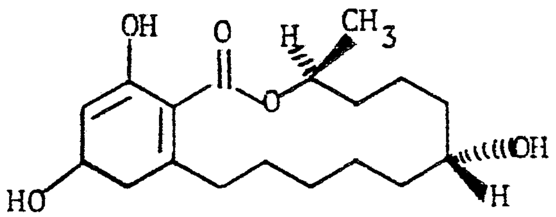

(2)

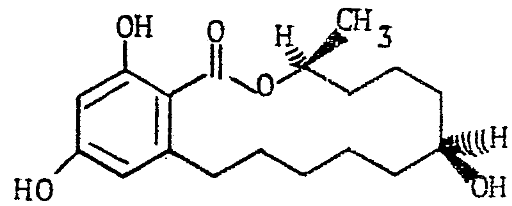

It is known that both diastereomers 1 and 2 are useful anabolic and estrogenic substances for oral and parenteral administration to animals in the manner disclosed e.g. in U.S. Pat. 3,239,345 (1966) and U.S. Pat. 4,069,339 (1978). Depending on the intended use for the compound, however, it may be preferred to employ one or the other diasteromer in a larger amount, or even exclusively.

It is the general trend, as is well-known to experts in the sector, to use pure diastereomer 1 (usually called "the higher melting diastereomer") as the anabolic, growth-promoting agent for meat producing animals, e.g. cattle, lamb and swine (see e.g. U.S. Pat. 4,069,339 (1978);"Anabolics in Animal Production" (Editors: E. Meissonier and J.M Vigneron, Symposium held at OIE, Paris, Feb. 15-17, 1983)).

It is also known, for example from P.H. Hidy et al. Adyan. Appln. Microbiol. 22 (1977) page 59, that the low melting diastereomer, also assigned as beta-zeranol, exhibits therapeutic effects in cases of difficience of estrogens in the women. It is registered under the name Ralone®.

Although a portion of "the higher melting diastereomer" (1) can be separated from the mixture with 2 by the procedures disclosed in U.S. Pat. 3,239,345 (fractional crystallization from isopropyl alcohol-water mixtures) this process is abandoned as uneconomical because of the poor yields on pure 1 after repeated crystallizations.

According to a more recent method, disclosed in U.S. Pat. 3.687 982 (1972) separation of the diastereomers 1 and 2can be effected by converting them to a mixture of their 7-acetate esters, separating the 7-acetate esters by fractional crystallization from a monohydric alcohol, e.g. from methanol, and hydrolyzing the separated esters to yield separated 1 and 2.

This method, although affording higher yield of 1 than the previously cited method has an obvious technological, and consequently economical, disadvantage of requiring three additional steps to separate the two diastereomers. Another drawback, which is common to both procedures, is that the separation is not nearly complete, and the remaining 1 still contains either a substantial amount of the other diastereomer (according to the U.S. Pat. 3,239,345) or detectible quantities of 7-acetate esters (according to the U.S. Pat. 3,687,982).

A mixture of diastereomers 1 and 2 can be obtained by first preparing and purifying zearalenone 3,-(R=H) i.e. the 7-oxo-analogue of 1 and 2, and then reducing either the unprotected compound 3, or properly a derivative in which the phenolic oxygen is protected, e.g. the o-benzyl-derivative 3a.

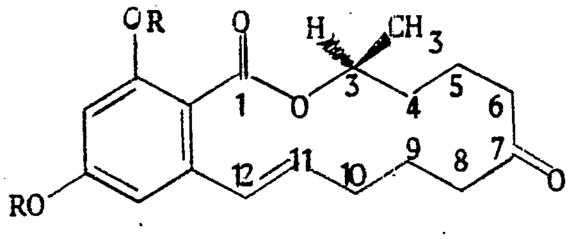

| | R |
|---|---|
| 3 | H |
| 3a | $CH_2Ph$ |

Reduction (hydrogenation) of 3 is repeatedly described in the literature, and is based on methods well-known to those skilled in the art. Hydrogenation over heterogeneous catalysts, like RaNi, Pd/C or Ni on a solid support, are known for example from U.S. Pat. 3,239,345 (1966); Ger. Offenlegungsschrift 21 11 606 (1971) and Ger. Offenlegungsschrift 22 53 467 (1972). In Ger. Offenlegungsschrift 23 28 605 (1973) hydrogenation over Pt/C modified with some weak organic acids is described, while in Ger. Offenlegungsschrift 21 11 618 (1971) where sodium hyroxide is used as modifier of RaNi a mixture 1/2in a ratio from 1:3 is obtained.

Hydrogenation with various achiral hydrides for reduction of ketonic carbonyl group in 3 is known from U.S. Pat. 3,704,249 (1972), while hydrogenation

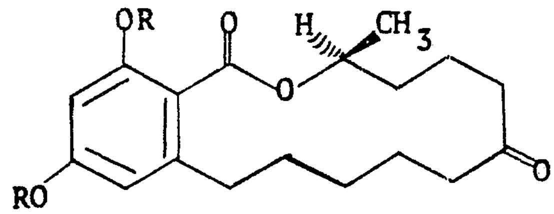

| | R |
|---|---|
| 4 | H |
| 4a | $CH_2Ph$ |

of 3, 3a, and their 11,12-dihydro-congeners 4,4a, is described in Ital. Pat. Appl. 83504 A/83 to CRC, Compagnia di Ricerca Chimica S.p.A.

In order to develop a most efficient method of the industrial scale, liquid-chromatography separation of the diastereomers 1 and 2, which method represents a substantial body of the invention claimed herein, the exact data about their chromatographic properties, conformation of the macrocyclic ring and absolute configuration at the chiral centres in the compound 1 were requested.

Prior to the invention described herein X-ray single crystal structure determinations have been published for two congeners of 1, both with trans-carbon-carbon double bonds, i.e. for the compounds 5 and 6 (5, zearalenol; W.H. Watson et al., Acta Cryst. B 38 (1982) 1037); 6, 4-hydroxyzearalenone, J.F. Taylor et al., Acta Cryst. B 32 (1976) 710).

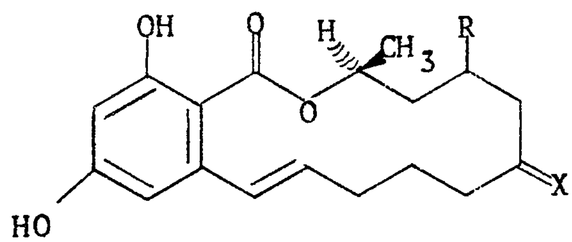

| | R | X |
|---|---|---|
| 5 | H | H,OH |
| 6 | OH | =O |

X-ray structure determination has been performed for one cis-isomer (cis-zearalenone, J.F. Griffin et al., Ann. Cryst. Assoc. Ser. 2 9 (1981) 35), and one O-substituted derivative (C. Ellestad et al., J. Org. Chem. 43 (1978) 2339).

Prior to the invention described herein neither the absolute configuration of "the high melting diastereomer" 1 nor its conformational properties were known. Moreover, some contradicting data appeared in the scientific literature. Thus, in the paper where separation of 1 and 2reverse phase thin-layer chromatography was described (D. Giron et al., J. High Res. Chromat., 1978, 67), as well as in the paper of T.H. Elsasser et al., J. Anim. Sci. 57 (1983) 443, wrong configurations (according to the present finding) are designed in the structural formulas of these diastereomers. Opposite configurations; i.e. the correct ones - according to the X-ray structural determination, are designed in the stereoformulas by A.M. Janski in "Anabolics in Animal Production" (Editors: E. Meissonier and J.M. Vigneron, Symposium held at OIA, Paris, February 15 - 17, 1985 pp. 443 - 546).

X-ray single crystal structure determination on 1, and then on its 7-acetyl ester derivative 7 was attempted.

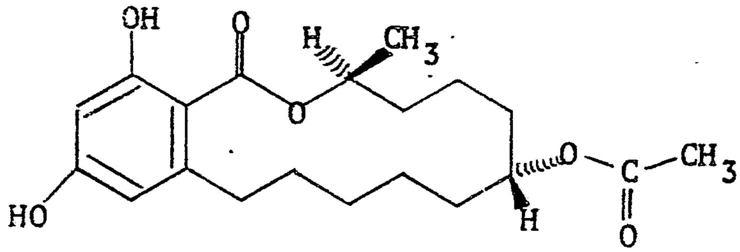

7

Compound 1 crystallyzes from various solvent systems (tetrahydrofurane-water, methanol-water, acetonitrile, tetrahydrofurane) as the twin crystals [space group P1, Z=2, triclinic; a=14.675 (11), b=11.574 (5), c=5.044 (2) Å; $\alpha$=89.96 (4)°, $\beta$=93.03 (4)°, $\gamma$=92.79 (3)°], thus precluding X-ray determination of the spatial structure of the molecule, and consequently of the three dimentional arrangement of the atoms around the chiral center at C(7). Therefore single crystals of its monoacetate 7 [space group $P2_12_12_1$, Z=4, ortho-rhombic were prepared; a=28.244 (2), B=9.503 (1), c=8.142 (1)]. Talbe 1 contains the values of the torsional angles in 7, calculated on the basis of the X-ray data and numbered as in the given formula.

Table 1

Angles of torsion for the compound 3S,7R-7, calculated on the basis of the data obtained from X-rays (the numbers of atoms are in accordance with the formula shown in this table). Om ( 1 -7 -26 -25 ) = -162.6

Om ( 1 -7 -26 -21 ) = 16.7
Om ( 2 -7 - 26 -25) = 19.3
Om ( 7 -2 -8 - 9 ) = -164.1
Om ( 2 -8 -9 -10 ) = 68.2
Om ( 8 -9 -10 -11 ) = -175.9
Om ( 9 -10 -11 -12) = 67.1
Om ( 10 -11 -12 -13) = 72.1
Om ( 11 -12 -13 -14) = -150.5
Om ( 12 -13 -14 -15) = 66.6
Om ( 13 -14 -15 -16) = 64.5
Om ( 14 -15 -16 -17) = -179.2
Om ( 15 -16 -17 -25) = -179.9
Om ( 16 -17 -25 -26) = - 93.1
Om ( 23 -5 -12 -11 ) = 102.4
Om ( 23 -5 -12 -13 ) = -133.6
Om ( 6 -23 -5 -12 ) = - 6.7

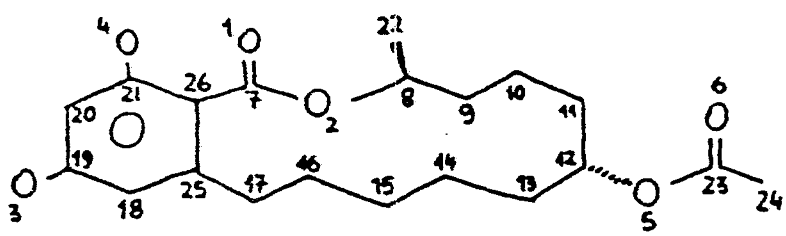

It has been found that compound 7, and consequently its deacetoxy congener 1, possess 3S,7R - absolute configuration, as shown in the formulas. This important finding allows a conclusion that the two larger substituents on the chiral centres C(3) and c(7), i.e. methyl and hydroxyl group, are trans -oriented, if the macrocyclic ring is flattened (1A). Obviously, in the diastereomer 2 ((3S,7S), the same two groups are cis-oriented (1B).

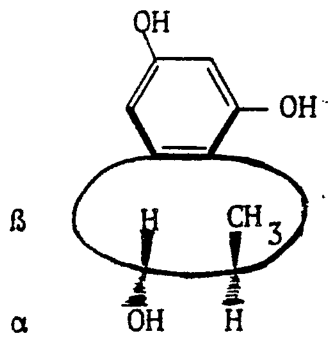

1

A

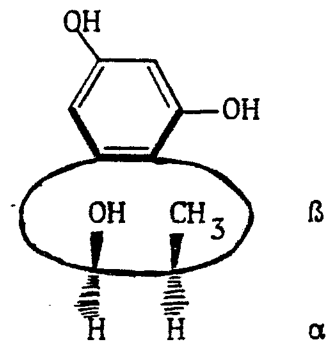

2

B

Schematic representation of the relative configurations of the diastereomers 3S,7R (A) and 3S, 7S (B).

Schematic representation of the flattened conformation of macrocyclic ring 1 and 2 is justified on the basis of circular dichroism (CD) data for both compounds, as well as for the dihydrate analogous to zearalenone 3, that is the compound 4. Reference is made to Fig. 1.

The CD spectra of these compounds were temperature dependant, indicating conformational stability around torsional angles 2-7-16-25 (19.3°) and 15-16-17-25 (-179.9°) (numbering from Table 1). This suggest that the stable (solid state) conformations, remained largely unchanged in the solution. For this conformation one can expect high preference for lipophilic interaction from the β-face, i.e. from the side where both lipophilic groups in the compound 1 are placed.

The structural and conformational findings allowed a conclusion, which constitutes the ultimate basis of the present invention. Lipophilic interaction of compound 1 with any lipophilic surface, as e.g: stationalary (reverse, lipophilic) phase in liquid chromatography should occur from the β-face and should be stronger than for the β-face of diastereomer 2, since both interaction, apolar, lipophilic groups (methyl and proton) in 1 are placed on the same (β) face. Consequently, in a reverse phase high pressure liquid chromatographic separation, compound 1 should have the higher retention times than compound 2, and should appear as the second from the reverse phase lipophilic chromatographic column. This assumption could experimentally be confirmed (see preparations) and makes the basis of a successful, technological separation of 1 from its mixtures with 2. Such chromatographic properties allow an easier optimization of the separation conditions, e. g. higher recovery and purity of 1 with the possibility of increasing the yield of the mixture separated daily using the technique of recycling.

Accordingly it is an object of the invention to provide a method for the separation of 3S,7R-3,4,5,6,7,8,9,10,11,12-decahydro-7,14,16-trihydroxy-3-methyl-1H-2-benzoxacyclotetradecin-1-one from its 3S,7S-diastereomer starting from mixtures of both.

It is a further object of the invention to provide a method for the separation of said diastereomers from mixtures, wherein the isomer 1 is present in quantities from 55 - 98 %.

It is likewise an object of the invention to provide a method for the separation of said diastereomers starting from mixtures of both which allows the separation of the two diastereomers in high purity.

## Summary of the Invention:

The present invention in its main embodiment comprises a method for the separation of 3S,7R-3,4,5,6,7,8,9,10,11,12-decahydro-7,14,16-trihydroxy-3-methyl-1H-2-benzoxacyclotetradecin-1-one ( 1) from its 3S,7S-diastereomer ( 2),

(1)

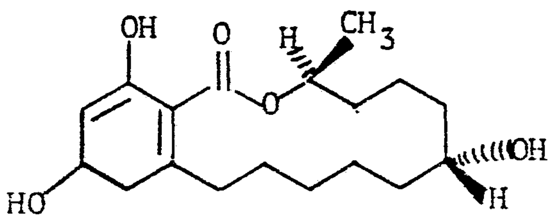

(2)

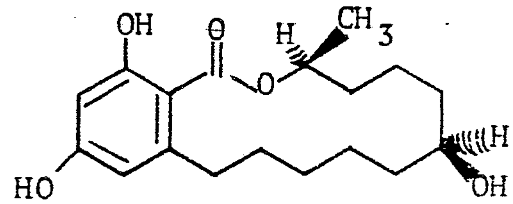

in which method

(a) a mixture of the two diastereomers (1) and (2) is dissolved in a solvent,

(b) the resulting solution is applied to a high pressure liquid chromatrograph comprising a reverse-phase column,

(c) the two diastereomers are eluted with a mixture of polar solvents and

(d) the diastereomers (1) and (2) are separately recovered by evaporating the solvent mixture at reduced pressure from those fractions that contain the diastereomer (1) in a ratio of at least 99 % and those fractions which contain the diastereomer (2) in a ratio of at least 98 %.

For the accomplishment of the invention the methods of High Pressure Liquid Chromatography (HPLC) can be used. The reverse-phase column of the chromatograph can be packed for example with packing materials selected from the group consisting of materials based on silica gel, aluminium oxide, cellulose or polyamides. According to one preferred embodiment of the invention the packing material consists of silica gel, which is lipophilically modified, e. g. Si-alkylated on the surface. Such packing materials or stationary phases are commercially known and available for example under Trade-names like Nucleosil ®, µ-Bondapak ® or Resolve ®. Such packing materials are generally characterized by an appendix, like $C_n$ in which n denotes the number of carbon atoms in the chain of the hydrocarbon phase chemically bonded to silica gel forming Si-C-bond.

According to one embodiment of the invention one of the following packing materials is used:

Novapak ® $C_{18}$ (particle size: 4µ)
µ-Bondapak ® $C_{18}$ (particle size: 10µ)
Resolve ® $C_{18}$ (particle size: 5µ)
Nucleosil ® $C_{18}$

According to another embodiment of the invention the columns containing the reverse-phase (RP) as the stationary phase are used in combination with a ternary solvent mixture, wherein one of the solvents is always water and the other two are organic solvents. For eluting the columns for example one of the following solvent mixtures with one of the given typical solvent ratios can be used:

water-methanol-acetonitrile (20:70:10)
water-tetrahydrofurane-methanol (20:20:60)
water-acetonitrile-isopropanol (50:45:5)
water-methanol-isopropanol (35:63:2)
water-tetrahydrofurane-isopropanol (50:40:10)
water-acetonitrile-acetic acid (44:55:1)
water-acetonitrile-tetrahydrofurane (30:60:10)
water-methanol-dioxane (40:40:20)
water-methanol-acetic acid (28:70:2)

Besides the mentioned organic solvents, other organic solvents can be used.

According to another preferred embodiment of the invention the mixture of diastereomers to be separated is dissolved in the smallest possible quantity of a solvent, like tetrahydrofurane or methanol.

The invention allows a new technological process for separating a mixture of diastereomers 1 and 2, which process comprises the following steps:

a. dissolving the mixed diastereomers 1 and 2 in a small quantity of a solvent, preferably in the smallest possible quantity such as tetrahydrofurane or methanol,

b. applying the resulting solution to a preparative liquid chormatograph with a reverse-phase (RP) column and eluting with a mixture of the polar solvents that ensure separation of the diastereomers 2 and 1,

c. recovery of the pure 1 and 2 by evaporating of the eluting solvent from different fractions under reduced pressure, the solvent mixture is re-used after control and adjustement to the original solvent ratio) crystallization of the pure compounds, collecting on a filtering device, washing and drying.

The advantages of the method of separation of 3S,7R-diastereomer 1 from 3S,7S-diastereomer 2 above described, in relation to the prior art procedures [disclosed in U.S. Pat. 3,239,345 (1966), and in U.S. Pat. 3,687,982 (1972)] are as follows:

a. nearly complete quantitative recovery of both 1 and 2 after an optimal number of cycles in the liquid chromatograph;

b. possibility of obtaining over 98 % pure 2, besides commercially important diastereomer 1 (not satisfied in the prior art procedures).

Figure 2 is a scheme of said process. A solution of 1 and 2 is supplied through the storage container (1) and injected by the injector (2) into the HPLC column (3). Reference number (4) denotes a detector and reference number (5) a recorder. During normal procedure valves 6,7 (or 8) are open and valves 9 and 10 are closed. During recycling the valves 9 and 10 are open and valves 6, 7 and 8 are closed. Solutions with separated 1 and 2 are collected in evaporating vessel (11), solvents are evaporated, residual solution cooled until crystallization of 1 or 2 is completed, and then crystals of pure 1 and 2 are collected on centrifuge (12).

Preparations:

To find out effective solvents for the elution of compounds 1 and 2 the following tests were performed:

A sample of a mixture of 1 and 2 (ratio about 60:40) was separated on reverse-phase thin layer chromatograph plates (RP-TLC Merck® RP18 $F_{244S}$ plates) using various solvent mixtures. The results are shown in the following Table 2.

Table 2

Solvent systems and $R_f$ values of 1 and 2 in chromatography experiments on thin layer in inverse phase (RP-TLC).

| N. | Solvent systems (vol : vol) | | $R_f$ Values | | $Rf_2/Rf_1$ |
|---|---|---|---|---|---|
| | | | 1 | 2 | |
| 1 | THF/MeCN | (60 : 40) | 0.38 | 0.43 | 1.13 |
| 2 | THF/$H_2O$ | (50 : 50) | 0.30 | 0.36 | 1.20 |
| 3 | MeCN/$H_2O$ | (65 : 35) | 0.42 | 0.48 | 1.14 |
| 4 | MeCN/$H_2O$ | (70 : 30) | 0.58 | 0.62 | 1.07 |
| 5 | MeCN/$H_2O$ | (60 : 40) | 0.45 | 0.50 | 1.11 |
| 6 | MeOH/$H_2O$ | (50 : 50) | 0.21 | 0.29 | 1.38 |
| 7 | MeHO/$H_2O$/i-PrOH | (35 : 63 : 2) | 0.35 | 0.43 | 1.23 |
| 8 | MeOH/$H_2O$ | (70 : 30) | 0.35 | 0.40 | 1.14 |
| 9 | THF/$H_2O$/MeOH | (10 : 30 : 60) | 0.45 | 0.48 | 1.07 |
| 10 | THF/$H_2O$/MeOH | (20 : 20 : 60) | 0.54 | 0.60 | 1.11 |
| 11 | THF/$H_2O$/MeOH | (20 : 40 : 40) | 0.25 | 0.30 | 1.20 |
| 12 | THF/$H_2O$/MeOH | (10 : 30 : 60) | 0.45 | 0.48 | 1.07 |
| 13 | MeCN/$H_2O$/AcOH | (44 : 55 : 1) | 0.30 | 0.44 | 1.47 |
| 14 | MeCN/MeOH/$H_2O$ | (10 : 70 : 20) | 0.40 | 0.61 | 1.27 |
| 15 | MeCN/MeOH/$H_2O$ | (40 : 30 : 30) | 0.40 | 0.43 | 1.08 |

On the basis of the above results, solvent mixtures 2,6,7,11,13 and 14 were used for the determination of the retention times ($R_t$ values) of 1 and 2 in analytical liquid chromatography on the reverse phase column with silanylated silica gel stationary phases of various types: 1) Nucleosil®$C_{18}$ (5μ) [dimensions of the column = 25 x 0.46 cm (Alltech)]; 2) μ-Bondapak® $C_{18}$ (10μ) [dimensions of the column = 30 x 0.39cm (Waters)]; 3) Resolve® $C_{18}$ (5μ) [dimensions of the column = 15 x 0.39 cm (Waters)], using instrumentation of the Perkin-Elmer type - series 2/2 with detector Spectrophotometer UV/VIS LC/55-B.

Relative retention times of compounds 1 and 2 are listed in Table 3. Determination of the peaks of compounds 1 and 2 was performed at 265 nm.

## Table 3

Solvent systems and $R_t$ values for 1 and 2 in analytical liquid chromatography on Nucleosil® $C_{18}$, Resolve® and µ-Bondapak® $C_{18}$ (speed of flow 1 ml/min.).

| N. | Solvent systems (vol : vol) | | $R_t$ values | | $R_{t1}/R_{t2}$ |
|---|---|---|---|---|---|
| | | | 1 | 2 | |
| Nucleosil® | | | | | |
| 1 | $MeOH/H_2O$ | (70:30) | 10.5 | 8.6 | 1.22 |
| 2 | $MeOH/H_2O$ | (80:20) | 12.0 | 10.7 | 1.12 |
| 3 | $H_2O/MeCN/THF$ | (30:60:10) | 8.7 | 7.0 | 1.27 |
| 4 | $MeCN/H_2O/i-PrOH$ | (45:50:5) | 6.7 | 5.4 | 1.19 |
| 5 | $H_2O/THF/i-PrOH$ | (50:40:10) | 5.45 | 4.85 | 1.12 |
| Resolve® | | | | | |
| 6. | $H_2O/MeCN/AcOH$ | (44:55:1) | 6.2 | 4.4 | 1.41 |
| 7 | $MeOH/MeCN/H_2O$ | (40:40:20) | 6.4 | 5.6 | 1.14 |
| Bondapak® | | | | | |
| 8 | $MeOH/THF/H_2O$ | (60:15:25) | 9.2 | 7.8 | 1.18 |
| 9 | MeOH/THF | (80:20) | 6.22 | 5.17 | 1.20 |
| 10 | $H_2O/MeOH/i-PrOH$ | (35:63:2) | 12.5 | 9.7 | 1.29 |
| 11 | $MeCN/H_2O$ | (55:45) | 14.2 | 11.8 | 1.20 |
| 12 | $MeOH/H_2O/MeCN$ | (40:20:40) | 6.0 | 5.4 | 1.11 |

On the basis of these results the following solvent systems were used for preparative scale separations:
water-methanol acetonitrile (20:70:10)
water-tetrahydrofurane-methanol (20:20:60)
water-acetonitrile-isopropanol (50:45:5)
water-methanol-isopropanol (35:63:2)
water-tetrahydrofurane-isopropanol (50:40:10)

water-acetonitrile-acetic acid (44:55:1)
water-acetonitrile-tetrahydrofurane (30:60:10)
water-methanol-dioxane (40:40:20)
water-methanol-acetic acid (28:70:2)

The following examples are to illustrate the method of the invention:

## Example 1

A mixture of diastereomers 1 and 2 (3.2 kg, ratio 65:35 determined on an analytical liquid chromatography instrument using a column filled with Nucleosil®$C_{18}$ and water-methanol-acetonitrile (20:70:10) as the eluant) prepared from zearalenone according to the method of Ital. Pat. Appl. 83504 A/83, was dissolved in 6 l of tetrahydrofurane and loaded on a column of the Waters Kiloprep instrument (dimensions of the radially compressed column: 15 x 60 cm; inverse phase $\mu$-Bondapak®$C_{18}$) and then eluted with a solvent mixture water-acetonitrile-tetrahydrofurane (30:60:10). The fractions containing mixture 1 and 2 were recycled until ca. 3 - 4 % of the mixture remained unseparated. Then the fractions containing pure 1 were collected, solvent evaporated to a small volume at reduced pressure (ca. 20 mm Hg), and at internal temperature ca. 40°C. When crystallization started evaporation was stopped, residual crystalline suspension was cooled under stirring to ca. 12°C for 2 - 3 hrs, separated crystalls were collected on filter, washed with ca. 3 l of water-methanol (9:1) mixture, and dried giving 980 g of 2 [98.1% purity, m.p. 156-158°C, $[\alpha]_D$ = + 38° (c = + 1.0, MeOH)]. Thereafter the fractions containing pure 1 were collected and compound 1 isolated in the same manner as described for 2 . 1960 g of 1 [99.2% purity, m.p. 180-182°C, $[\alpha]_D$ = + 46.2° (c = 1.0, MeOH] were obtained.

## Example 2

A mixture of diastereomers 1 and 2 (2 kg, ratio 74:26 determined as described in Example 1), prepared from zearalenone according to the method of Italian patent application 83504 A/83 was dissolved in 6 l tetrahydrofurane and loaded on a column of the Waters Kiloprep instrument (dimensions of the radially compressed column: 15 x 60 cm; inverse phase $\mu$-Bondapak®$C_{18}$) and then eluted with a solvent mixture water-methanol-acetonitrile (20:70:10).

The fractions containing mixtures 1 and 2 were recycled until ca. 3 - 4 % of the mixture remained unseparated. The fractions containing pure 2 were collected, solvent evaporated to a small volume at reduced pressure, and at ca. 35 - 40°C, crystalline residual suspension was cooled for 2 - 3 hrs, and separated crystalls were collected on filter, washed with a small quantity of water, and dried to give 490 g of 2 (98.5 % purity, m.p. 156 - 157°C $[\alpha]_D$ = + 39° (c = 1.0, MeOH)]. Thereafter the fractions containing pure 1 were collected, and compound 1 isolated in the same manner as described for 2. It was obtained 1360 g of 1 [99 % purity, m.p. 180 - 182°C, $[\alpha]_D$ = + 46.3° (c = 1.0, MeOH)].

## Example 3

A mixture of the diastereomers 1 and 2 (20 g, ratio 1:2 ca. 60:40) was separated by high pressure liquid chromatography, using a Waters-Prep LC/500 A System, with cartridges prepacked with Nucleosil®$C_{18}$, and varying the eluting solvent mixture. The results are shown in the following Table 4.

Table 4

Solvent systems, $R_t$ values and quantities separated of 1 and 2 in preparative liquid chromatography on Nucleosil®$C_{18}$ (20 g, mixture 1:2 = 60:40) (speed of flow 200 ml/min.).

| N. | Solvent systems (vol : vol) | Rt [min] | | gr 1 (purity %) | gr 2 (purity %) |
|---|---|---|---|---|---|
| | | 1 | 2 | | |
| 1 | $H_2O$/THF/MeOH (20 : 20 : 60) | 3.5 | 3.0 | 11.06 (99.2) | 7.6 (98.5) |
| 2 | $H_2O$/MeOH/MeCN (20 : 70 : 10) | 5.5 | 4.5 | 11.3 (99.0) | 7.8 (98.0) |
| 3 | $H_2O$/THF/i-PrOH (550 : 40 : 10) | 7.4 | 6.3 | 11.5 (99.3) | 7.7 (98.2) |
| 4 | $H_2O$/MeCN/THF (30 : 60 : 10) | 7.3 | 5.1 | 11.4 (99.5) | 7.5 (98.3) |

Example 4

A mixture of diastereomers 1 and 2 (260 g, ratio ca.70:30) was dissolved in 800 ml of tetrahydrofurane and loaded on the column of a Waters Prep LC/500 A System containing cartridges prepacked with Nucleosil®$C_{18}$ (30 x 5.7 cm). The mixture was eluted with a water-methanol-isopropanol (35:63:2) mixture, which was recycled until the fractions with pure 2 were separated, which on evaporation of the solvent mixture to about 0.5 l, colling and filtering off the crystalline precipitate afforded 62 g of pure 2[98.7 % purity, m.p. 155 - 158°C, $[\alpha]_D$ = +38.8° (c = 1.0, MeOH)], while the separated fractons with pure 1, after the same elaboration as above afforded 145 g of pure 1 [99 % purity, m.p. 180 - 182°C, $[\alpha]_D$ = /46.2° (c = 1.0, MeOH)].

Example 5

A mixture of diastereomers 1 and 2 (260 g, ratio ca. 80:20) was dissolved in 800 ml of tetrahydrofurane and separated as described in Example 4, but using solvent system water-acetonitrile-acetic acid (44:55:1) for elution. Collected fractions with pure 2 were evaporated at reduced pressure and at 40°C, giving 38 g of 2 (98.8% purity). The same isolation procedure was repeated with 1, giving 182 g of 1 (99 % purity). For both products the principal impurity (50 - 220 ppm) representing the C (7)-monoacetylated derivative.

Example 6

A mixture of diastereomers 1 and 2 (10 kg, ratio 60:40) determined as described in the Example 1) was loaded on the column of a Waters Kiloprep instrument (dimentions of the radially compressed column: 15 x 60 cm, inverse phase μ-Bondapak®$C_{18}$). Following the same procedure as described in the Example 1, 3.8 kg of the pure diastereomer 2 (98 % purity, m.p. 156-158°C), and 5.4 kg of the pure diastereomer 1 (99 % purity, m.p. 181 - 182°C) were obtained.

**Claims**

1. The method for the separation of 3S,7R-3,4,5,6,7,8,9,10,11,12-decahydro-7,14,16-trihydroxy-3-methyl-1H-2-benzoxacyclotetradecin-1-one (1) from its 3S,7R-diastereomer (2),

(1)

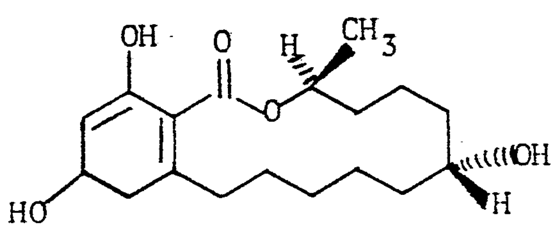

(2)

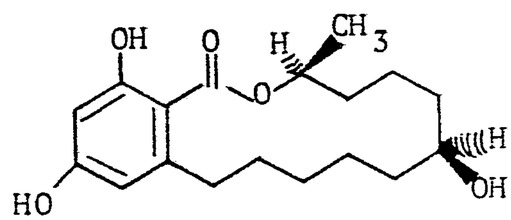

characterized in that

(a) a mixture of the two diastereomers (1) and (2) is dissolved in a solvent,

(b) the resulting solution is applied to a high pressure liquid chromatograph comprising a reverse-phase column,

(c) the two diastereomers are eluted with a mixture of polar solvents and

(d) the diastereomers (1) and (2) are separately recovered by evaporating the solvent mixture at reduced pressure from those fractions that contain the diastereomer (1) in a ratio of at least 99 % and those fractions which contain the diastereomer (2) in a ratio of at least 98 %.

2. The method according to claim 1, characterized in that in step (a) as the solvent tetrahydrofurane or methanol is used.

3. The method according to claim 1, characterized in that in step (c) a ternary solvent mixture, wherein one of the solvents is water, is used.

4. The method according to claim 3, characterized in that one of the following ternary solvent mixtures is used:

water-methanol-acetonitrile ;
water-tetrahydrofurane-methanol;
water-acetonitrile-isopropanol;
water-tetrahydrofurane-isopropanol;
water-acetonitrile-acetic acid;
water-acetonitrile-tetrahydrofurane;
water-methanol-dioxane;
water-methanol-acetic acid.

5. The method according to claim 1, characterized in that in step (b) a high pressure liquid chromatograph comprising a reverse-phase column is used, which is packed with materials based on silica gel, aluminium oxide, cellulose or polyamide.

6. The method according to claim 1, characterized in that in step (c) separation of the mixture 1 and 2 - (wherein 1 is present from 55 - 99%) is performed by continuous recycling the fractions that contain less than 99 % of 1, and continuous product (1 ) separation with a purity equal or higher than 99 %, whereby the principal impurity in the product thus obtained is the second diastereomer 2.

7. The method according to claim 1, characterized in that in step (b), contemporaneously with the separation of the principal product 1, separation of 2, with purity equal or higher than 98 %, is achieved.

8. The method according to claim 1, characterized in that in step (c) evaporation of the solvent mixture from the fractions that contain pure 1 and 2 is performed at reduced pressure and at a temperature below 40°C.

9. The method according to claim 5, characterized in that as the packing material a silanylated silica gel is used.

10. The method according to claim 1, wherein in the step (d), evaporation of the solvent mixture from fractions that contain pure 1 and 2 is performed until the pure compounds start to crystallize, whereafter the crystallizing mixture, is cooled and the pure product is filtered off.

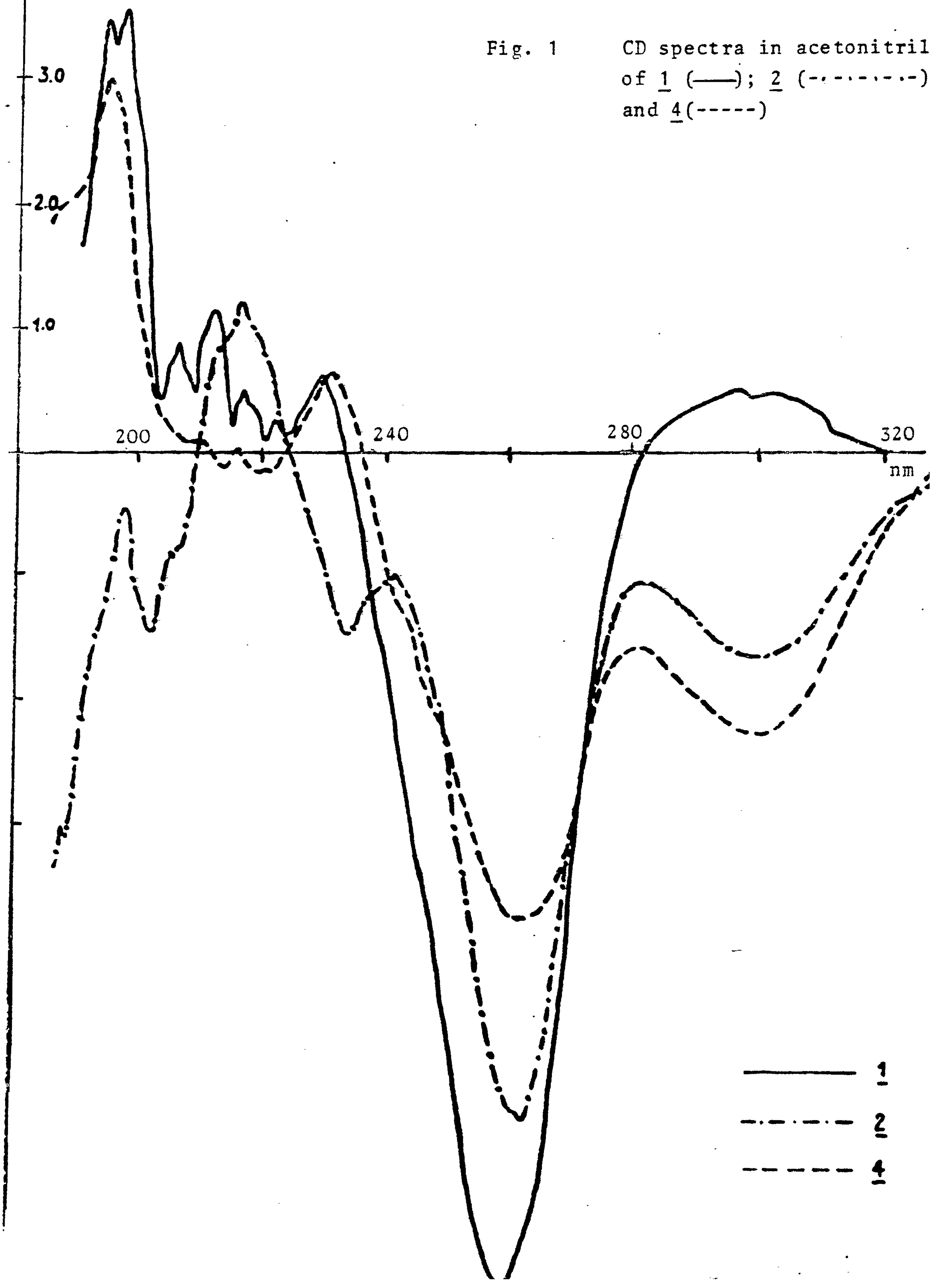


Fig. 1 CD spectra in acetonitril of 1 (——); 2 (-·-·-·-·-) and 4(-----)

Fig. 2

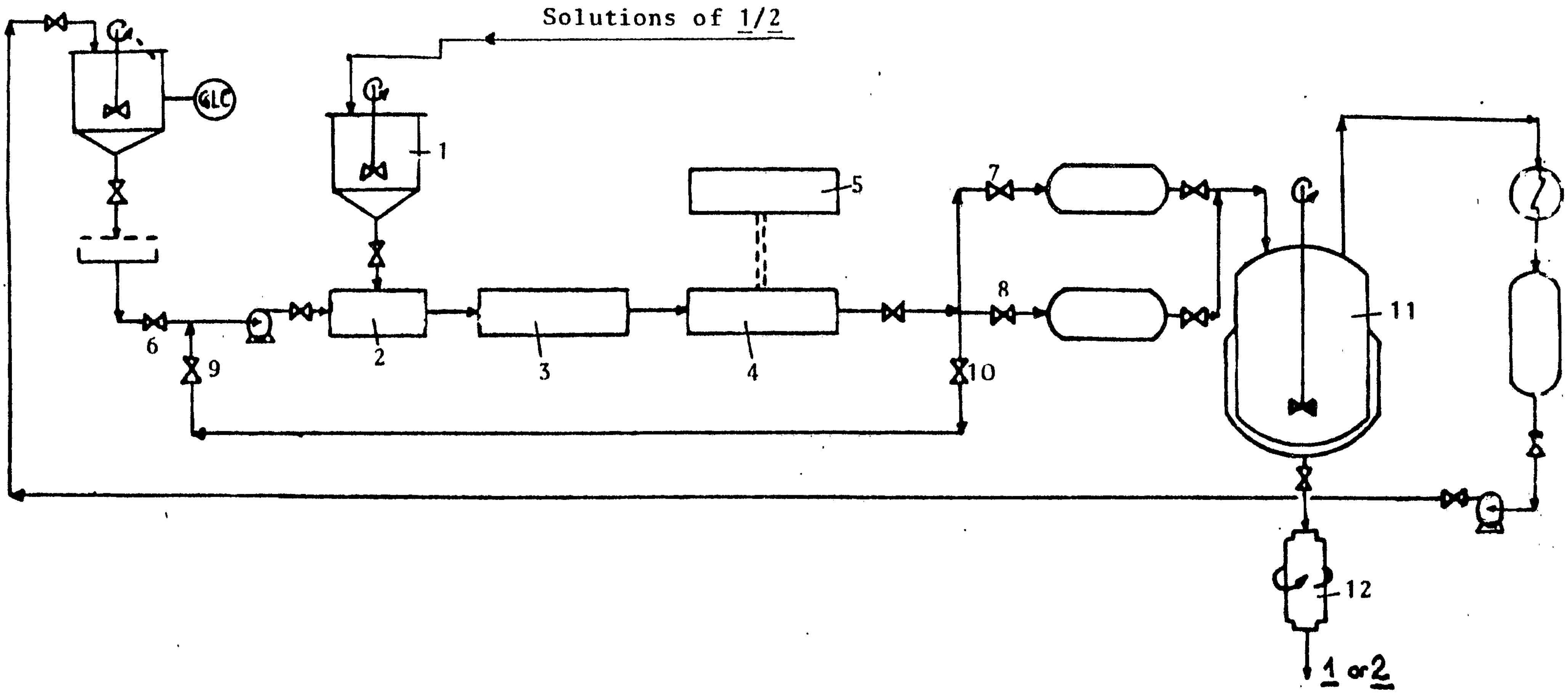

European Patent Office

# EUROPEAN SEARCH REPORT

Application number

EP 86107692.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR - A1 - 2 571 372 (PANMEDICA)<br>* Claims 1-4 *<br>-- | 1-5 | C 07 D 313/00<br>B 01 D 15/08<br>/A 23 K 1/16<br>A 61 K 31/365 |
| D,A | US - A - 3 687 982 (YOUNG)<br>* Abstract *<br>-- | 1,10 | |
| A | US - A - 4 052 414 (PETERS)<br>* Abstract *<br>---- | 1 | |

TECHNICAL FIELDS SEARCHED (Int. Cl.4)

C 07 D 313/00

The present search report has been drawn up for all claims

| Place of search | Date of completion of the search | Examiner |
|---|---|---|
| VIENNA | 04-02-1987 | LUX |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document